# DEMANDE DE BREVET EUROPEEN

(11) **EP 0 692 249 A1**
(43) Date de publication de la demande: **17.01.1996**
(21) Numéro de dépôt: 95401638.2
(22) Date de dépôt: 07.07.1995
(51) Int. Cl.: A61K 31/19, A61K 47/12

(54) **Composés comportant une ou plusieurs fonctions oximes, stabilisés en solution aqueuse, leur procédé d'obtention, et leur utilisation en tant que médicaments**

(30) Priorité: 13.07.1994 FR 9408769
(71) Demandeur: LABORATOIRES JACQUES LOGEAIS Société dite:, F-92130 Issy-Les-Moulineaux (FR)
(72) Inventeur: Legeai, Jacky, F-92160 Antony (FR); Fedynec, Richard, F-78730 Longvilliers (FR); Cavaillon, Pascal, F-92500 Rueil Malmaison (FR)
(74) Mandataire: Grosset-Fournier, Chantal Catherine

(57) **Abrégé**

La présente invention a pour objet tout composé comportant au moins une fonction oxime, stabilisé en solution aqueuse, et tel qu'obtenu par addition d'un agent anionique à ladite solution aqueuse comprenant le composé susmentionné.

L'invention vise également les compositions pharmaceutiques comprenant un composé stabilisé tel que décrit ci-dessus, notamment du Ximoprofène stabilisé en solution aqueuse.

## Description

La présente invention a pour objet des composés comportant une (ou plusieurs) fonction(s) oxime(s) stabilisé(s) en solution aqueuse.

L'invention concerne également un procédé d'obtention de ces composés stabilisés, ainsi que des compositions pharmaceutiques comprenant ces derniers.

De nombreux composés comportant une (ou plusieurs) fonction(s) oxime(s), et plus particulièrement ceux comportant une (ou plusieurs) fonction(s) ionisable(s) en plus de ces fonctions oximes, sont instables en solution aqueuse. Cette instabilité se traduit par une hydrolyse de la fonction oxime en fonction aldéhyde ou cétone.

A titre d'illustration d'oxime particulièrement instable en solution aqueuse, on peut citer le Ximoprofène (DCI).

Le Ximoprofène, ou acide 2-[4-(3-hydroxyiminocyclohexyl)phényl] propionique, est une molécule connue pour ses propriétés anti-inflammatoires et analgésiques, et est plus particulièrement décrite dans la demande de brevet français n° 2 243 680.

Il serait particulièrement intéressant d'utiliser ce composé en thérapeutique sous forme d'une préparation aqueuse dans les manifestations inflammatoires de l'organisme accessibles à un traitement par voie locale.

Or, le Ximoprofène, en solution à des pH compatibles avec une application locale, en particulier cutanée, ne présente pas une stabilité chimique suffisante pour permettre une telle utilisation du Ximoprofène en application locale.

En effet, après quelques jours en solution aqueuse à pH 5,8 ou 7, le Ximoprofène subit une hydrolyse importante de la fonction oxime conduisant à un dérivé cétonique, selon le schéma réactionnel suivant:

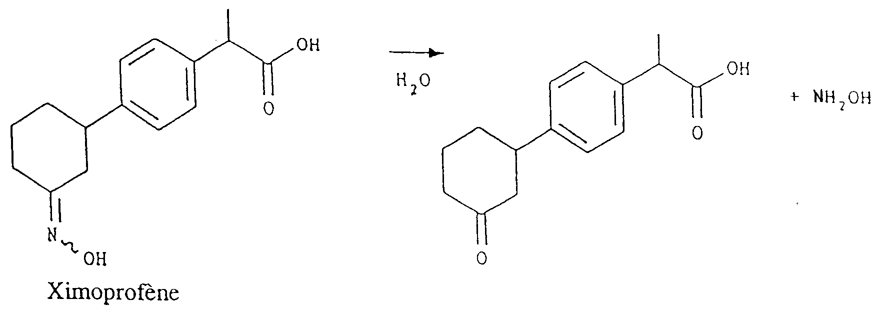

Le Ximoprofène en solution à 0,5%, conservé 5 jours à 45°C est hydrolysé à près de 50% dans les conditions de pH susmentionnées.

Il est généralement reconnu qu'un composé sensible à l'hydrolyse peut être protégé par un système micellaire non ionique (J. Pharm. Pharmacol. (1991), 43: 237-241). Le Ximoprofène n'est pas protégé par ces systèmes puisque 3 à 12% de cétone apparaissent dès la mise en solution du composé dans l'eau en présence de différents tensioactifs non ioniques tels que Tween 80*, Oranax CG110*, Simusol 68* ou Crémophar RH40* (*: marque déposée). La même dégradation du principe actif est observée en présence d'agents cationiques tels que Amonyl DM* ou Antanox*.

Le but de la présente invention est celui d'obtenir la stabilisation en solution aqueuse de ceux des composés comportant une (ou plusieurs) fonction(s) oxime(s), et plus particulièrement ceux susceptibles d'être substitués par une (ou plusieurs) fonction(s) ionisable(s), qui, notamment en raison de la nature de leur environnement stérique, ne sont pas stables en solution aqueuse.

Un autre but de la présente invention est celui de fournir des compositions aqueuses contenant des composés comportant une (ou plusieurs) fonction(s) oxime(s) stabilisés, et plus particulièrement des compositions pharmaceutiques comprenant des composés susmentionnés en tant que principe actif, stabilisés en solution aqueuse, notamment du Ximoprofène ainsi stabilisé.

Un autre but de la présente invention est celui de fournir un procédé pour la stabilisation des composés comportant une (ou plusieurs) fonction(s) oxime(s) en solution aqueuse.

La présente invention a pour objet tout composé comportant au moins une fonction oxime, stabilisé en solution totalement ou partiellement aqueuse, et tel qu'obtenu par addition d'un agent anionique à ladite solution aqueuse comprenant le composé susmentionné, ledit composé étant instable en solution totalement ou partiellement aqueuse, notamment lorsqu'il n'est pas mis en solution avec ledit agent anionique.

Par composé comportant au moins une fonction oxime, et étant instable en solution totalement ou partiellement aqueuse, on entend tout composé dont la fonction oxime, ou l'une au moins de ses fonctions oximes, est susceptible d'être transformée par hydrolyse en fonction cétone ou aldéhyde, dans une proportion telle qu'au moins 10% de la quantité totale dudit composé mise en solution aqueuse simple, se trouve être ainsi transformé après une période d'environ 2 à environ 5 jours à 45°C, ou encore pendant une période d'au moins un mois à 37°C.

Par composé stabilisé en solution aqueuse comprenant au moins une fonction oxime selon l'invention, on entend tout composé dont la fonction oxime, ou l'une au moins de ses fonctions oximes, est susceptible d'être transformée par hydrolyse en fonction cétone ou aldéhyde, dans une proportion inférieure à 10%, et avantageusement inférieure à environ 5% de la quantité totale dudit composé mise en solution aqueuse, pendant une période d'au moins un mois à 37°C, notamment dans des proportions dudit composé en solution aqueuse d'environ 0,1% à environ 2% en poids par volume.

Avantageusement, le pH de la solution totalement ou partiellement aqueuse dans ce qui précède et ce qui suit, est compris entre environ 5 et environ 7.

Par l'expression "solution totalement ou partiellement aqueuse", dans ce qui précède et ce qui suit, on entend toute solution aqueuse comprenant, le cas échéant, un solvant non aqueux miscible au solvant aqueux, tel qu'un solvant alcoolique, ou un solvant non miscible, pouvant former une émulsion ou un additif pouvant former un gel tel que le Sepigel (marque déposée).

Parmi les composés susceptibles d'être stabilisés selon l'invention, on peut citer les oximes présentant une structure linéaire ou cyclique, et plus particulièrement celles comportant une (ou plusieurs) fonction(s) ionisable(s), c'est-à-dire une fonction susceptible de former un sel.

Parmi les composés cycliques, on peut citer plus particulièrement ceux présentant la formule (I) suivante: 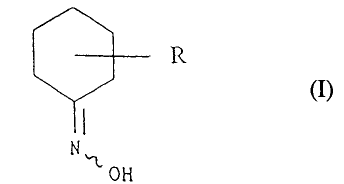
R représentant:
- une chaîne hydrocarbonée de 1 à 10 atomes de carbone, le cas échéant substituée, notamment par un groupe alkyle de 1 à 5 atomes de carbone, ladite chaîne comportant soit dans sa partie terminale, soit sur un de ses substituants, une (ou plusieurs) fonction(s) ionisable(s), notamment une ou plusieurs fonction(s) -COOH, ou
- un cycle, notamment aromatique, le cas échéant substitué, notamment par un groupe alkyle de 1 à 5 atomes de carbone, ledit cycle étant substitué soit directement, soit par l'intermédiaire de l'un de ses substituants, par une (ou plusieurs) fonction(s) ionisable(s), notamment par une ou plusieurs fonction(s) -COOH.

L'invention concerne plus particulièrement à titre de composé stabilisé selon l'invention, le Ximoprofène dont la formule est indiquée ci-dessus.

L'agent anionique utilisé pour stabiliser les composés selon l'invention est avantageusement choisi parmi les stéarates, notamment parmi les stéarates de potassium, de sodium, d'ammonium ou de triéthanolamine.

Un agent anionique particulièrement préféré dans le cadre de la présente invention est le stéarate de triéthanolamine.

L'invention a également pour objet toute composition comprenant au moins un composé comportant une (ou plusieurs) fonction(s) oxime(s), tel que décrit ci-dessus, en solution totalement ou partiellement aqueuse, et un agent anionique, choisi notamment parmi ceux définis ci-dessus.

Avantageusement, la proportion de composé dans la composition susmentionnée est d'environ 0,1% à environ 2% en poids par volume, tandis que la proportion d'agent anionique dans cette même solution est d'environ 1% à environ 3% en poids par volume.

L'invention a plus particulièrement pour objet une composition telle que définie ci-dessus, comprenant du Ximoprofène et du stéarate de triéthanolamine en solution aqueuse, notamment dans les proportions indiquées ci-dessus.

L'invention a également pour objet toute composition pharmaceutique comprenant un composé stabilisé en solution aqueuse, tel que décrit ci-dessus, ou une composition susmentionnée, en mélange avec un véhicule pharmaceutiquement acceptable.

Les compositions pharmaceutiques selon l'invention, sont avantageusement destinées à l'application topique, et se présentent notamment sous forme de gel, patch, crème, gouttes, ou encore de solutions pour pulvérisations et nébulisations.

L'invention concerne également l'utilisation d'un agent anionique, choisi notamment parmi ceux définis ci-dessus, pour l'obtention d'un composé comportant au moins une fonction oxime et stabilisé en milieu totalement ou partiellement aqueux.

L'invention vise également l'utilisation d'un composé stabilisé, tel que décrit ci-dessus, ou d'une composition susmentionnée, pour l'obtention d'un médicament destiné au traitement des inflammations, notamment en rhumatologie, traumatologie, phlébologie, dermatologie, stomatologie, ophtalmologie, ainsi que pour le traitement des oedèmes et des inflammations de la sphère O.R.L.

L'invention concerne également un procédé de stabilisation d'un composé comportant au moins une fonction oxime, tel que décrit ci-dessus, en solution totalement ou partiellement aqueuse, caractérisé en ce que l'on effectue un mélange dudit composé en solution aqueuse avec un agent anionique choisi notamment parmi ceux définis ci-dessus.

La présente invention sera davantage illustrée dans ce qui suit à l'aide d'un exemple de stabilisation du Ximoprofène en solution aqueuse.

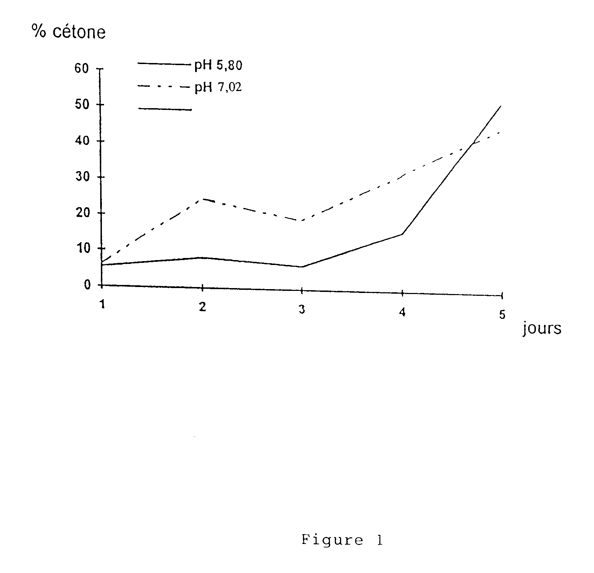

La cinétique d'hydrolyse du Ximoprofène en solution aqueuse simple est donnée dans le tableau I qui suit, et est représentée sur la figure 1.

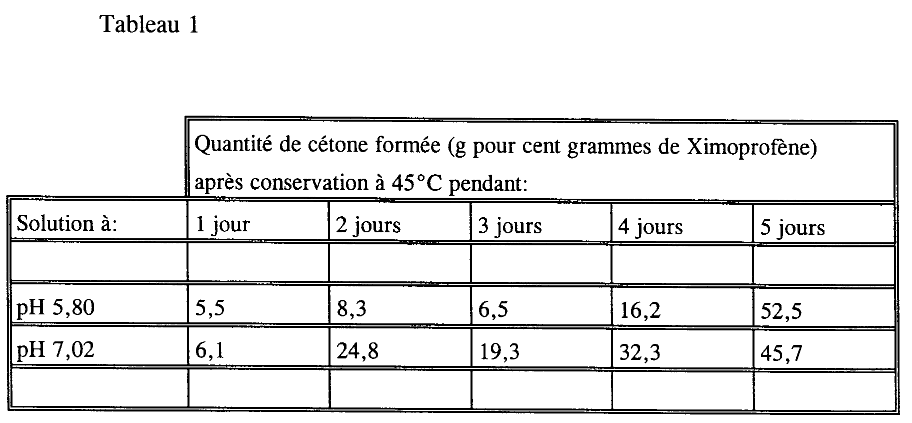

Légende de la figure 1: le temps de conservation de la solution du Ximoprofène est indiqué en jours en abscisse, le pourcentage de cétone formée dans la solution est indiqué en ordonnée; la courbe représentée par _ .. _ correspond à la cinétique d'hydrolyse à pH 7,02, et la courbe représentée par correspond à la cinétique d'hydrolyse à pH 5,80.

Comme le montre le tableau II ci-après, en présence d'agents anioniques tels que des stéarates, la stabilité du Ximoprofène en solution aqueuse se trouve considérablement augmentée.

En effet, le Ximoprofène en solution aqueuse à des concentrations de 0,1 à 1% (masse/volume), en présence de stéarate de potassium ou de sodium ou d'ammonium ou de préférence de stéarate de triéthanolamine dans des proportions de 1 à 3% (masse/volume) ne subit pas d'hydrolyse importante après une conservation de 20 jours à 45°C.

**Tableau 2**

| Stabilité du Ximoprofène en solution aqueuse à 0,5% (m/m) | | | |
|---|---|---|---|
| Concentration du stéarate en % m/v | Teneur en cétone (g pour 100 g de Ximoprofène) après conservation pendant 20 jours à 45°C en présence du stéarate de: | | |
| | Potassium | Sodium | Triéthanolamine |
| 1,0 | 4,5 | 3,4 | |
| 1,5 | 4,9 | 4,9 | 3,8 |
| 2,0 | 7,6 | 6,6 | 3,6 |
| 3,0 | 9,2 | 12,7 | 3,8 |

### Mode opératoire

Du stéarate de triéthanolamine (6 g) est dissous dans 573 g d'eau déminéralisée préalablement chauffée à 60°C. Une agitation à 300 tours/minute est nécessaire pendant 30 à 60 minutes.

A la solution refroidie à 20°C, le Ximoprofène (3 g) est ajouté en poudre fine. Une solution blanche et homogène est obtenue après une agitation d'au moins 6 heures à 300 tours/minute.

Le digluconate de chlorhexidine (0,06 g) est ensuite dissous dans cette solution, puis le Sépigel 305 (marque déposée) (18 g) est ajouté.

L'agitation douce 300 tours/minute est progressivement augmentée pour atteindre 800 tours/minute après 45 minutes.

Un gel blanc et homogène (600 g) est ainsi obtenu, dans lequel le principe actif est suffisamment stable.

Ximoprofène gel à 0,5% - Etude de stabilité

Résultats après conservation à température ambiante (TA), 37°C et 45°C jusqu'à 3 mois; tableau 3.

La méthode de dosage de la cétone formée dans la solution aqueuse de Ximoprofène, est réalisée par chromatographie liquide haute performance sur colonne C18. Le système éluant utilisé est:
- acétonitrile: 15%,
- sulfate d'ammonium 0,1M (ramené à pH 7 par l'ammoniaque) -: 85%.

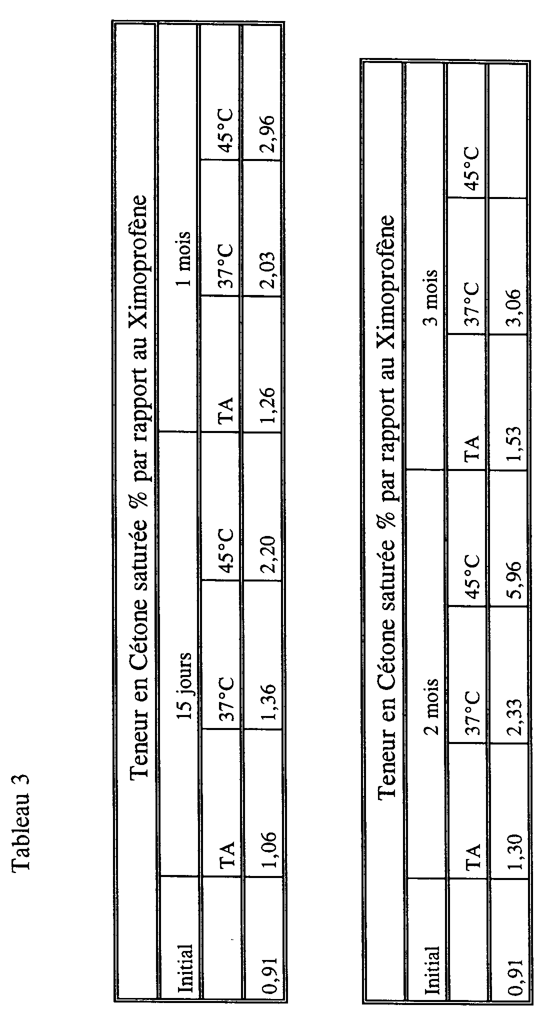

## Revendications

1. Composé comportant au moins une fonction oxime, stabilisé en solution totalement ou partiellement aqueuse, et tel qu'obtenu par addition d'un agent anionique à ladite solution aqueuse comprenant le composé susmentionné, ledit composé étant instable en solution totalement ou partiellement aqueuse, notamment lorsqu'il n'est pas mis en solution avec ledit agent anionique.

2. Composé stabilisé en solution aqueuse selon la revendication 1, caractérisé en ce que la fonction oxime, ou l'une au moins de ses fonctions oximes, est susceptible d'être transformée par hydrolyse en fonction cétone ou aldéhyde, dans une proportion inférieure à 10%, notamment inférieure à 5%, de la quantité totale dudit composé mise en solution aqueuse, pendant une période d'au moins un mois à 37°C, notamment dans des proportions dudit composé en solution aqueuse d'environ 0,1% à environ 2% en poids par volume.

3. Composé stabilisé selon la revendication 1 ou la revendication 2, caractérisé en ce qu'il présente une structure linéaire ou cyclique, et comporte le cas échéant une (ou plusieurs) fonction(s) ionisable(s),.

4. Composé stabilisé selon l'une des revendications 1 à 3, caractérisé en ce qu'il présente la formule (I) suivante: 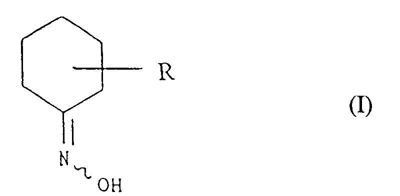 R représentant:
- une chaîne hydrocarbonée de 1 à 10 atomes de carbone, le cas échéant substituée, notamment par un groupe alkyle de 1 à 5 atomes de carbone, ladite chaîne comportant soit dans sa partie terminale, soit sur un de ses substituants, une (ou plusieurs) fonction(s) ionisable(s), notamment une ou plusieurs fonction(s) -COOH, ou
- un cycle, notamment aromatique, le cas échéant substitué, notamment par un groupe alkyle de 1 à 5 atomes de carbone, ledit cycle étant substitué soit directement, soit par l'intermédiaire de l'un de ses substituants, par une (ou plusieurs) fonction(s) ionisable(s), notamment par une ou plusieurs fonction(s) -COOH.

5. Composé stabilisé selon l'une des revendications 1 à 4, caractérisé en ce qu'il est représenté par le Ximoprofène de formule

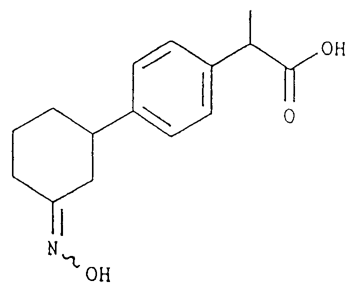

6. Composé stabilisé selon l'une des revendications 1 à 5, tel qu'obtenu par mise en présence dudit composé avec un agent anionique choisi parmi les stéarates, notamment parmi les stéarates de potassium, de sodium, d'ammonium ou de triéthanolamine.

7. Composé stabilisé selon l'une des revendications 1 à 6, caractérisé en ce que le stéarate est un stéarate de triéthanolamine.

8. Composition caractérisée en ce qu'elle comprend un composé selon l'une des revendications 1 à 6, en solution totalement ou partiellement aqueuse, et un agent anionique.

9. Composition selon la revendication 8, caractérisée en ce que le composé est tel que défini dans l'une des revendications 1 à 5.

10. Composition selon la revendication 8 ou la revendication 9, caractérisée en ce que l'agent anionique est tel que défini dans la revendication 6 ou la revendication 7.

11. Composition selon l'une des revendications 8 à 10, caractérisée en ce que la proportion de composé dans ladite solution est d'environ 0,1% à environ 2% en poids par volume, tandis que la proportion d'agent anionique dans cette même solution est d'environ 1% à environ 3% en poids par volume.

12. Composition pharmaceutique, caractérisée en ce qu'elle comprend un composé stabilisé selon l'une des revendications 1 à 7, ou une composition selon l'une des revendications 8 à 11, en mélange avec un véhicule pharmaceutiquement acceptable.

13. Composition pharmaceutique selon la revendication 12, caractérisée en ce qu'elle est destinée à l'application topique, et se présente notamment sous forme de gel, patch, crème, gouttes, ou encore de solutions pour pulvérisations et nébulisations.

14. Utilisation d'un agent anionique choisi notamment parmi ceux définis dans la revendication 6 ou la revendication 7, pour l'obtention d'un composé comportant au moins une fonction oxime et stabilisé en milieu totalement ou partiellement aqueux.

15. Utilisation d'un composé stabilisé selon l'une des revendications 1 à 7, ou d'une composition selon l'une des revendications 8 à 11, pour l'obtention d'un médicament destiné au traitement des inflammations, notamment en rhumatologie, traumatologie, phlébologie, dermatologie, stomatologie, ophtalmologie, ainsi que pour le traitement des oedèmes et des inflammations de la sphère O.R.L.

16. Procédé de stabilisation d'un composé comportant au moins une fonction oxime, en solution totalement ou partiellement aqueuse, notamment d'un composé tel que défini dans l'une des revendications 1 à 5, caractérisé en ce que l'on mélange ledit composé en solution aqueuse avec un agent anionique choisi notamment parmi ceux définis dans les revendications 6 et 7.
